# EUROPÄISCHE PATENTSCHRIFT

(11) **EP 2 760 985 B1**
(45) Veröffentlichungstag und Bekanntmachung des Hinweises auf die Patenterteilung: **15.11.2017**
(21) Anmeldenummer: 11770396.7
(22) Anmeldetag: 30.09.2011
(51) Int. Cl.: C12C 1/10, C12C 1/135, C12C 1/02, C12C 1/027

(54) **VORRICHTUNG UND VERFAHREN ZUM WEICHEN, KEIMEN, DARREN, FERMENTIEREN UND/ODER KOMBINATIONEN DARAUS VON KORN**
DEVICE AND METHOD FOR STEEPING, GERMINATING, KILNING, FERMENTING, AND/OR COMBINATIONS THEREOF, GRAIN
DISPOSITIF ET PROCÉDÉ POUR LE TREMPAGE, LA GERMINATION, LE TOURAILLAGE, LA FERMENTATION DE GRAINS ET/OU DES COMBINAISONS DE CES OPÉRATIONS

(43) Veröffentlichungstag der Anmeldung: 06.08.2014
(73) Patentinhaber: Bühler AG, 9240 Uzwil (CH)
(72) Erfinder: GÖRLITZ, Frank-Otto, 38116 Braunschweig (DE); SILBERMANN, Kai, 38116 Braunschweig (DE); BISCHOFF, Wolf-Eckart, 38176 Wendeburg (DE)
(74) Vertreter: Vossius & Partner Patentanwälte Rechtsanwälte mbB
(86) Internationale Anmeldenummer: PCT/EP2011/067143
(87) Internationale Veröffentlichungsnummer: WO 2013/044984

(56) Entgegenhaltungen:
- DE-A1- 1 642 651
- DE-A1- 2 523 709
- GB-A- 1 122 807
- GB-A- 1 538 177

## Beschreibung

Die Erfindung betrifft eine Vorrichtung sowie ein Verfahren zum Weichen, Keimen, Darren, Fermentieren und / oder Kombinationen daraus von Korn nach den Merkmalen der Oberbegriffe der unabhängigen Patentansprüche. Ferner wird die Verwendung der Vorrichtung, ein Kit und ein Verfahren zur betriebsfertigen Installation der Vorrichtung beansprucht.

Vorrichtungen zum Weichen und Keimen sind im Zusammenhang mit Braugerste bekannt, wobei durch Erhöhung des Wassergehalts beim Weichen die Keimruhe überwunden wird und die Braugerste zu Keimen beginnt. Das hierbei erhaltene Grünmalz wird anschliessend gedarrt beziehungsweise getrocknet und hierbei lagerfähiges Malz erhalten. Solche Vorrichtungen werden als Mälzereien bezeichnet. Solche Einweich- und Keimungsschritte finden zudem bei weiteren Pflanzenarten Anwendung wie weitere Getreidearten nebst Braugerste, Hülsenfrüchte, Pseudogetreide und Ölsaaten. Im Sinne der Anmeldung werden Getreide, Pseudogetreide, Ölsaaten und Hülsenfrüchte und / oder deren Bestandteile und / oder deren Mischungen unter dem Begriff Korn zusammengefasst.

Aus dem Stand der Technik sind Mälzereien bekannt, bei welchen mittels temperierter und befeuchteter Luft beispielsweise in so genannten Keimkasten nach Saladin auf einer Platte mit Öffnungen, auch Lochblech genannt, gekeimt wird. Das Anbringen eines Lochblechs erlaubt es, Wasser zum Einweichen oder Luft während dem Keimen beziehungsweise Darren mit dem zu verarbeitenden Korn durch das Lochblech in Kontakt zu bringen, ohne dass das zu verarbeitende Korn zwischen Prozessschritten wie dem Keimen und Darren beispielsweise in einen weiteren Prozessraum überführt werden muss. Das beim Einweichen eingesetzte Wasser wird während des Prozesses verschmutzt und muss als Abwasser betrachtet werden. Aus diesem Grund ist es unter anderem wünschenswert, den Wasserverbrauch gering zu halten.

Dokument DE 101 12 406 offenbart eine Vorrichtung zum Einweichen und Belüften während des Einweichens von Gerste. Ein Lochblech ist in dieser Vorrichtung derart ausgestaltet, dass ein Durchtritt von Wasser in den dem Prozessraum abgewandten Bereich unterhalb des Lochblechs vermieden wird, womit der Wasserverbrauch gering gehalten werden kann. Das Lochblech ist lediglich so ausgestaltet, das Gase aus dem Raum unterhalb des Lochblechs in den Prozessraum gelangen können. Die Umsetzung des Lochblechs dieser Vorrichtung ist technisch anspruchsvoll und daher aufwendig in der Herstellung

DE1642651 offenbart eine einstöckige Keimvorrichtung bsp. nach Art eines Saladinkatens für Gerste und andere zu mälzende Körnerfrüchte (Gut), mit einem nach oben abschließbaren Aufnahmebehälter, einer zum Tragen des Gutes über dem Behälterboden angeordneten Horde, einer über der Horde mündenden Luftabführ- bzw. Luftzuführleitung, sowie einer zwischen Behälterboden und Horde mündenden absperrbaren Luftzuführ bzw. Luftabführleitung.

Aus WO 2005/083050 ist eine Vorrichtung zum Weichen von Gerste bekannt, wobei unter einem Boden mit Perforationen beziehungsweise ein Lochblech direkt an Durchgänge zum Durchführen von Wasser über diese Durchgänge anschliesst. Durch diese direkte Wasserzuführung direkt an das Lochblech kann der Wasserverbrauch gering gehalten werden. Eine solche Vorrichtung ist allerdings konstruktiv aufwendig.

Dokument GB2405075 offenbart ein Verfahren zum Einweichen von Gerste mittels drei Einweichbehältern. Die Einweichbehälter weisen im Konus ein Lochblech auf, auch Konusweiche genannt, durch welche direkt Wasser zu- beziehungsweise Abwasser abgeführt werden kann. Durch die direkte Wasserzuführung kann der Wasserverbrauch gering gehalten werden. Eine Leitung zur Zufuhr eines Gases während des Einweichens ist über dem Lochblech angeordnet, was konstruktiv umständlich ist.

Es ist deshalb eine Aufgabe der vorliegenden Erfindung, eine Vorrichtung sowie ein Verfahren zum Weichen, Keimen, Darren, Fermentieren und / oder Kombinationen daraus bereitzustellen, welche die beschriebenen Nachteile des Standes der Technik überwindet. Insbesondere soll eine konstruktiv einfachere Vorrichtung sowie ein Verfahren zum Weichen, Keimen, Darren, Fermentieren und / oder Kombinationen daraus bereitgestellt werden mit verringertem Wasserverbrauch insbesondere während des Weichens des Korns.

Erfindungsgemäss wird diese Aufgabe gemäß der Patentansprüche 1-14 gelöst.

Die erfindungsgemässe Vorrichtung zum Weichen, Keimen, Darren, Fermentieren und / oder Kombinationen daraus umfasst einen Behälter mit mindestens einer im Behälter anbringbaren Platte mit mindestens einer Öffnung, insbesondere zur Zufuhr und / oder Abfuhr von, insbesondere flüssigem und / oder gasförmigem, Fluid, wobei der Behälter zumindest eine, insbesondere im Wesentlichen rechteckige, Grundfläche, auf der Grundfläche insbesondere im Wesentlichen vertikal, d.h. im Wesentlichen in einem 90° Winkel, angeordnete insbesondere zueinander parallele Seitenflächen, auf der Grundfläche insbesondere im Wesentlichen vertikal angeordnete insbesondere zueinander parallele Stirnflächen sowie eine auf mindestens einer Seitenfläche und / oder Stirnfläche insbesondere im Wesentlichen horizontal, d.h. im Wesentlichen parallel zur Grundfläche, angeordnete Deckfläche zur Bildung eines Prozessraums umfasst und wobei auf das Innenmass bezogen eine Seitenlänge der Stirnfläche 2 bis 2,5 Meter, insbesondere 2,345 Meter, entspricht.

Diese Anordnung entspricht einer leicht zu realisierenden und platzsparenden Konstruktion einer Vorrichtung zum Weichen, Keimen, Darren, Fermentieren und Kombinationen daraus von Korn.

Im Sinne der vorliegenden Erfindung sind die Seitenflächen und / oder die Stirnflächen die Wände, die Grundfläche die Bodenplatte und die Deckfläche die Deckplatte des Behälters.

Bevorzugt entspricht bei bestimmungsgemässem Gebrauch der Vorrichtung eine im Wesentlichen waagerecht verlaufende Seitenlänge der Stirnfläche 2 bis 2,5 Meter, insbesondere 2,345 Meter.

Bevorzugt sind die Seitenflächen länger ausgestaltet als die Stirnflächen. Damit erhält insbesondere der Behälter vorteilhaft die Quaderform eines Containers, welcher sich besonders gut für den Transport eignet.

Bevorzugt weisen Grundfläche, Seitenflächen, Stirnflächen sowie die Deckfläche auf der dem Prozessraum zugewandten Seite eine lebensmitteltaugliche Oberfläche und / oder Beschichtung auf. Im Sinne der vorliegenden Anmeldung wird unter dem Begriff lebensmitteltauglich verstanden, dass das zu verarbeitende Korn nach dem Kontakt mit der Oberfläche oder Beschichtung noch für den menschlichen Verzehr geeignet ist.

Vorzugsweise ist die Platte mit mindestens einer Öffnung horizontal beabstandet und im Wesentlichen parallel zur Grundfläche insbesondere an mindestens einer Seitenfläche und / oder Stirnfläche anbringbar. Die Platte mit mindestens einer Öffnung kann deckungsgleich zur Grundfläche und / oder Deckfläche sein.
Der Bereich oberhalb dieser Platte auf der der Grundfläche abgewandten Seite bildet im Wesentlichen den Prozessraum. Bevorzugt ist die Platte mit mindestens einer Öffnung klappbar angeordnet, so dass ein Zugang auf der dem Prozessraum abgewandten Seite zum Raum zwischen Grundfläche und Platte geschaffen wird zur Erleichterung der Reinigung dieses Raums. Besonders bevorzugt ist die Platte manuell schwenkbar, insbesondere aufklappbar, zu einer parallel zu einer längeren Seite einer Seitenfläche verlaufenden Drehachse und an der gegenüberliegenden Seitenfläche blockierbar, insbesondere abstützbar. Zur Abstützung kann beispielsweise eine Auflagefläche vorgesehen sein. In der Betriebsstellung ist die Platte an der gegenüberliegenden Seitenfläche blockiert.

Vorzugsweise ist die Platte mit mindestens einer Öffnung im Wesentlichen parallel zur Grundfläche und / oder Deckfläche im Behälter angeordnet. Ein Abstand (a) der Grundfläche zur Platte mit mindestens einer Öffnung steht bevorzugt zu einem Abstand (b) der Platte mit mindestens einer Öffnung zu einer maximalen Schütthöhe des Korns in einem Längenverhältnis von bevorzugt 1 : 2 bis 1 : 3, noch bevorzugter 1 : 2,25 bis 1 : 2,75, am meisten bevorzugt 1 : 2,4 bis 1 : 2,5.

In einem derartigen Längenverhältnis des Abstands (a) zum Abstand (b) ist einfache Vorrichtung realisiert, mittels welcher gleichzeitig der Wasserverbrauch insbesondere für das Weichen des Korns gering gehalten werden kann.

Im Sinne der Erfindung wird unter einer maximalen Schütthöhe des Korns die Höhe verstanden, bis zu welcher eine Umwälzung, d.h. ein Auflockern über die gesamte Schütthöhe der Kornschicht, aufgrund der Dimension, d.h. der Höhe, der Wendewerkzeuge bei bestimmungsgemässem Gebrauch erfolgen kann. Beispielsweise kann auch die maximale Schütthöhe aus dem maximal zulässigen Druckverlust in einer durch eine Einrichtung zur Luftzufuhr, insbesondere während des Keimens, erzeugten Strömung durch die Schütthöhe der Korns bestimmt werden; beispielsweise kann aus diesem maximal zulässigen Druckverlust, der durch die Schütthöhe des Korns erzeugt wird, die Höhe der Wendewerkzeuge bestimmt werden.

Bevorzugt kann die Grundfläche als Platte mit mindestens einer Öffnung ausgestaltet sein. Eine solche Anordnung hat den Vorteil, dass ein besonders grosses Volumen zur Aufnahme von Korn im Behälter zur Verfügung steht.

Vorzugsweise ist in und / oder angrenzend an die Platte mit mindestens einer Öffnung eine Abfuhreinrichtung zum Abführen des Korns aus dem Behälter angeordnet sowie eine im Behälter anbringbare und insbesondere bewegbare Wendeeinrichtung zum Wenden des zu verarbeitenden Korns enthalten. Die Wendeeinrichtung ist im Weiteren vorzugsweise als Einrichtung zum Fördern des Korns zur Abfuhreinrichtung ausgebildet, insbesondere zum Entleeren des Behälters, womit die Wendeeinrichtung zumindest eine Umwälzfunktion zum Wenden und eine Ausräumfunktion zum Fördern annehmen kann.

Im Sinne der vorliegenden Offenbarung wird unter einer Ausräumfunktion der Wendeeinrichtung verstanden, dass die Wendeeinrichtung so einstellbar ist, um als Einrichtung zum Fördern des Korns verwendet zu werden.

Bevorzugt umfasst die Wendeeinrichtung zumindest mindestens eine Wendeschnecke und / oder Wendespirale und einen Antrieb für die Wendeschnecke oder Wendespirale.

Vorzugsweise weist die zumindest eine Wendeschnecke oder Wendespirale ein Vollgewinde auf. In der Ausräumfunktion steht die Wendeschnecke oder Wendespirale vorzugsweise still und ist als Mittel zum Fördern des Korns insbesondere blockierbar.

Im Sinne der Anmeldung wird unter blockierbar verstanden, dass die Wendeschnecken oder Wendespiralen so kontrollierbar sind, um eine Änderung der Position der Wendeschnecken oder Wendespiralen untereinander im Wesentlichen zu verhindern. Dies kann beispielsweise durch Steuerung und /oder Regelung des Antriebs erfolgen.

Im Weiteren kann auch ein flächiges Element an dem der Platte zugewandten Ende der Wendeschnecke oder Wendespirale angeordnet sein, welches in einer Position der Wendeschnecken oder Wendespiralen einen Schieber bildet zum Fördern des Korns. Vorzugsweise ist die Positionierung der flächigen Elemente zu einem Schieber mittels eines Positionssensors einstellbar beziehungsweise gezielt positionierbar. Bevorzugt sind zumindest zwei Wendeschnecken oder Wendespiralen mit einem flächigen Element auf dem der Platte zugewandten Ende in der Vorrichtung angeordnet, die in einer Position einen Schieber bilden zum Fördern des Korns.

Die Ausgestaltung einer Wendeeinrichtung zum Fördern des Korns zu einer Abfuhreinrichtung dient einer leicht zu realisierenden und platzsparenden Konstruktion der erfindungsgemässen Vorrichtung.

Bevorzugt ist die Abfuhreinrichtung bei bestimmungsgemässem Gebrauch fest, d.h. nicht bewegbar, in der Vorrichtung angeordnet. Dies vereinfacht vorteilhaft die Konstruktion der erfindungsgemässen Vorrichtung.

Im Weiteren ist die Abfuhreinrichtung bevorzugt im Prozessraum insbesondere benachbart an eine Seitenfläche und / oder Stirnfläche angebracht. Durch diese Anordnung wird das Fördern des Korns zur Abfuhreinrichtung hin vereinfacht. Im Weiteren ist die Abfuhreinrichtung bevorzugt im Raum zwischen der Grundfläche und der Platte mit mindestens einer Öffnung angeordnet.

Bevorzugt umfasst die Abfuhreinrichtung zumindest einen in Förderrichtung, d.h. in Richtung der Abfuhreinrichtung, abschnittsweise konisch zulaufenden Abfuhrbehälter und / oder einen, insbesondere im konisch zulaufenden Abfuhrbehälter angeordneten, Schneckenförderer. Im Weiteren ist vorzugsweise der konisch zulaufende Abfuhrbehälter komplementär zum Querschnitt des Schneckenförderers ausgebildet, d.h. der Abfuhrbehälter ist an der Position des Schneckenförderers komplementär zu dessen rundem Querschnitt ausgebildet. Damit wird eine möglichst vollständige Entleerung des verarbeiteten Korns aus dem Behälter erleichtert.

An der Abfuhreinrichtung kann im Weiteren eine, insbesondere verschliessbare, Ausräumklappe und / oder ein Flachschieber anbringbar sein, um den Zugang des Korns zum Abfuhrbehälter freizugeben beziehungsweise abzusperren. Durch das Anbringen einer Ausräumklappe beziehungsweise eines Flachschiebers wird vorteilhaft eine den Abfuhrbehälter absperrende Schranke zur Verhinderung des Austritts des zu verarbeitenden Korns während den Prozessschritten gebildet, welche zum Entleeren des Korns über den Abfuhrbehälter freigegeben werden kann. Die Ausräumklappe und / oder der Flachschieber können manuell, pneumatisch oder hydraulisch bedienbar sein. Bevorzugt ist die Ausräumklappe in Richtung des konisch zulaufenden Abfuhrbehälters klappbar. Vorzugsweise ist der Flachschieber mittels einer Einrichtung auf einer dem Prozessraum abgewandten Aussenfläche des Behälters insbesondere manuell bedienbar.

Im Sinne der Erfindung wird unter konisch eine sich verengende Querschnittsfläche im Wesentlichen senkrecht zur Eintrittsöffnung für Korn in den Abfuhrbehälter verstanden.

Vorzugsweise liegt ein Winkel α, welcher zumindest abschnittweise zwischen einer durch eine Eintrittsöffnung der Abfuhreinrichtung gebildeten Fläche und der konischen Zulaufrichtung zum Abfuhrbehälter aufgespannt wird, im Bereich von 10 bis 80°, bevorzugt von 30 bis 70°, noch bevorzugter von 50 bis 60°.

Der Winkel α ist damit derart gewählt, dass der Abstand a zwischen Grundfläche und Platte mit mindestens einer Öffnung einerseits möglichst gering ist und andererseits ein weitestgehendes Fördern des Korns zur Abfuhreinrichtung, insbesondere zum Schneckenförderer, mittels der Wendeeinrichtung gewährleistet ist, insbesondere zur weitestgehenden Entleerung des verarbeiteten Korns aus dem Behälter sowie ein Schneckenförderer durch einen möglichst grossen Querschnitt eine hohe Austragskapazität gewährleistet.

Im Weiteren ist vorzugsweise die Wendeeinrichtung zur Abfuhreinrichtung hin bewegbar, insbesondere verschiebbar. Dies hat den Vorteil, dass das Korn möglichst weitgehend anhand der Abfuhreinrichtung aus dem Behälter entleert werden kann.

Vorzugsweise sind die Seitenflächen und / oder Stirnflächen bei bestimmungsgemässem Gebrauch fest, d.h. nicht verschiebbar, auf der Grundfläche angeordnet, womit die notwendige Stabilität des Behälters während des Transports gewährleistet ist.

Vorzugweise sind die Wendeschnecken oder Wendespiralen höhenverstellbar im Wesentlichen parallel und / oder antiparallel zur Richtung der Schwerkraft bei bestimmungsgemässem Gebrauch ausgebildet. Im Weiteren können die Wendeschnecken oder Wendespiralen zu einer im Wesentlichen zur Stirnwand parallel verlaufenden Drehachse schwenkbar sein, insbesondere von einer im Wesentlichen parallel zur Schwerkraft verlaufenden Vertikalstellung in eine Horizontalstellung.

Dies erlaubt vorteilhaft, auf einfache Weise Manipulationen an den Wendewerkzeugen vorzunehmen.

Vorzugsweise umfasst die Wendeeinrichtung zumindest eine an mindestens einer, insbesondere verstärkten, Seitenfläche und / oder Stirnfläche angebrachte Führung sowie einen Antrieb zum Verschieben der Wendeeinrichtung entlang der Führung.

Bevorzugt ist die Führung als Leiste mit einer Triebstockverzahnung ausgestaltet. Im Weiteren kann die Wendeeinrichtung entlang der Leiste mittels einer Führungsrolle verschiebbar sein, wobei die Führungsrolle mittels einer Welle, insbesondere eine Kraftübertragungswelle mit dem Antrieb zum Verschieben der Wendeeinrichtung kraftschlüssig verbunden ist.

Vorzugsweise weist mindestens eine, bevorzugt zwei Seitenfläche(n) und / oder Stirnfläche(n) des Behälters zumindest eine, insbesondere im Wesentlichen komplementäre, Aussparung zumindest zur teilweisen Aufnahme der Wendeeinrichtung, insbesondere der Wendeschnecken oder Wendespiralen, auf.

Ein solche Aussparung hat den Vorteil, dass bei Anbringen der Abfuhreinrichtung angrenzend an eine Seitenfläche und / oder Stirnfläche die Wendeeinrichtung nahe an die Abfuhreinrichtung verschoben werden kann, um eine weitestgehende Entleerung des verarbeiteten Korns aus dem Behälter zu erzielen, ohne dass ein grosser Anteil Korn durch die Wendeeinrichtung über die Abfuhreinrichtung gefördert wird und anschliessend nicht mehr durch die Wendeeinrichtung in die Abfuhreinrichtung förderbar ist, wie dies beispielsweise bei der Verwendung von benachbart angeordneten Wendespiralen der Fall sein kann.

Vorzugsweise ist an einer Seitenfläche und / oder Stirnfläche eine, insbesondere krümmbare, Energieführungseinrichtung, insbesondere ein Kabelkanal oder eine Energieführungskette, angeordnet und mit der Wendeeinrichtung wirkverbunden, wobei in der Energieführungseinrichtung insbesondere Kabel zur Energieversorgung der bewegbaren Wendeeinrichtung aufnehmbar sind.

Bevorzugt ist die Energieführungseinrichtung bei bestimmungsgemässem Gebrauch in einer Ebene im Wesentlichen parallel zur Deckfläche krümmbar.

Vorzugsweise ist die Energieführungseinrichtung entlang zweier im Wesentlichen paralleler Führungsschienen krümmbar, wobei sich die Führungsschienen insbesondere im Wesentlichen parallel zu den Seitenflächen erstrecken. Vorzugsweise sind die Führungsschienen im Wesentlichen in einer Ebene parallel zur Deckfläche zueinander beabstandet angeordnet. Die Führungsschienen sind vorzugsweise in einem Raum zwischen der maximalen Schütthöhe und der Deckfläche angeordnet. Die zweite Führungsschiene ist vorzugsweise an einer Seitenfläche und / oder Deckfläche angeordnet.

Bevorzugt erstreckt sich die erste Führungsschiene im Wesentlichen entlang der Länge der Seitenflächen. Vorzugsweise weist die zweite Führungsschiene im Wesentlichen eine Länge im Bereich von 40% bis 60% der Länge der ersten Führungsschiene auf und erstreckt sich insbesondere von einer

Stirnfläche bis zur Mitte einer längeren Seite einer Seitenfläche.

Im Weiteren weist die Energieführungseinrichtung insbesondere an den Enden bevorzugt mindestens zwei Fixierungen im Behälter auf, wobei eine Fixierung an der Wendeeinrichtung und eine weitere Fixierung, insbesondere im Bereich der Mitte einer längeren Seite der Seitenfläche, an der Deckfläche, der Seitenfläche oder der zweiten Führungsschiene angebracht ist.

Diese Art der Anordnung der Energieführungseinrichtung weist den Vorteil auf, dass das Volumen zur Aufnahme von Korn zwischen der Platte mit mindestens einer Öffnung und der Deckfläche kaum reduziert wird.

Bevorzugt ist an der Wendeeinrichtung eine Benetzungseinrichtung anbringbar. Vorzugsweise ist das Korn mit Wasser benetzbar. Besonders bevorzugt sind, insbesondere gelöst im Wasser, Zusatzstoffe in den Behälter zuführbar.
Dies hat den Vorteil, dass entsprechend den Prozessanforderungen Wasser oder beispielsweise Vitamine und / oder Mineralien zuführbar sind zur Erhöhung der Qualität des Endprodukts.

Vorzugsweise kann die Benetzungseinrichtung im Weiteren als Reinigungseinrichtung ausgestaltet sein, welche insbesondere eine Cleaning-in-Place Reinigung (Reinigung vor Ort) erleichtert. Besonders bevorzugt ist eine Reinigungseinrichtung als separate Einrichtung ausgebildet, insbesondere zusätzlich zur Benetzungseinrichtung.

Vorzugsweise ist insbesondere entlang einer Achse an einer Verteileinrichtung mindestens eine Öffnung, besonders bevorzugt mehrere Öffnungen, zur Zuführung von Korn in den Behälter durch die zumindest eine Öffnung vorhanden.

Im Weiteren wird unter Verteileinrichtung eine Einrichtung zur verteilten Zufuhr des zu verarbeitenden Korns in den Behälter an mindestens einer Position verstanden. Durch die verteilte Zufuhr des zu verarbeitenden Korns durch verschiedene Öffnungen verringert sich vorteilhaft ein Verteilaufwand des Korns innerhalb des Prozessraums.

Vorzugsweise ist die Verteileinrichtung im Wesentlichen entlang einer Achse insbesondere an der Deckfläche angebracht, welche im Wesentlichen parallel zu den Seitenflächen des Behälters verläuft. Durch das Anbringen der Verteileinrichtung an der Deckfläche kann das zu verarbeitende Korn mittels der Wirkung der Schwerkraft ohne zusätzlich notwendige Fördermittel in den Prozessraum gelangen.

Bevorzugt umfasst die Verteileinrichtung zumindest eine Fördereinrichtung, um das zu verarbeitende Korn an die gewünschte Position zu fördern. Die Fördereinrichtung erleichtert ein kontrolliertes Fördern des zu verarbeitenden Korns.

Vorzugsweise ist die Verteileinrichtung und / oder die Fördereinrichtung steuerbar und / oder regelbar.

Insbesondere umfasst die Verteileinrichtung regelbare und / oder steuerbare, insbesondere getaktete, Absperrschieber auf zum Öffnen und / oder Schliessen der Öffnungen. Dies hat den Vorteil einer weiteren Verringerung des Verteilaufwands des Korns innerhalb des Prozessraums und einer gleichmässigeren Verteilung des Korns.

Vorzugsweise umfasst die Vorrichtung mindestens eine Einrichtung zur Zufuhr eines, insbesondere konditionieren, Fluids, insbesondere für Wasser zum Weichen des Korns und / oder für ein Gas.

Die Einrichtung zur Zufuhr von Wasser kann als separate Einrichtung ausgestaltet sein von der Einrichtung zur Zufuhr eines Gases. Unter dem Begriff Gas wird beispielsweise für die Prozessschritte Keimen und Darren konditionierte Luft verstanden. Besonders bevorzugt erfolgt die Zufuhr des Gases beziehungsweise der konditionierten Luft mittels einer Fluidleitung unterhalb der Platte mit mindestens einer Öffnung.

Bevorzugt sind die Grundfläche, Seitenflächen, Stirnflächen und / oder Deckfläche des Behälters oder alle möglichen Kombinationen daraus doppelwandig wie beispielsweise die folgenden Kombinationen: Grundfläche, Seitenflächen und Stirnflächen; Deckfläche, Seitenflächen, Stirnflächen; Grundfläche und Seitenflächen; Grundflächen und Stirnflächen; Seitenflächen und Stirnflächen; Grundfläche und Deckfläche; Seitenflächen und Deckflächen; Stirnflächen und Deckfläche. Im Sinne der Erfindung ist unter dem Begriff doppelwandig zu verstehen, dass die Wände der Flächen zumindest teilweise voneinander beabstandet sind.

Dies hat den Vorteil, dass der Behälter insbesondere gegenüber äusseren Temperatureinflüssen und insbesondere gegenüber Temperaturschwankungen besser isoliert wird, wobei während des Keimens gegenüber Kälte und während des Darrens gegenüber Wärmeverlusten isoliert wird. Optional kann in der Beabstandung zwischen den beiden Wänden eine zusätzliche Isolierschicht vorhanden sein.

Als weiterer Vorteil stellt ein doppelwandiger Behälter einen verbesserten Schutz des Behälterinneren gegenüber Beschädigungen beispielsweise beim Transport der Vorrichtung dar.

Vorzugsweise ist die Vorrichtung zweiteilig ausgestaltet, wobei ein erstes Vorrichtungselement zumindest den Behälter und eine zweites Vorrichtungselement zumindest eine Einrichtung zur Zufuhr eines Gases, insbesondere konditionierte Luft in den Behälter umfasst. Durch eine Auftrennung der Vorrichtung vereinfacht sich der Transport für die zwei jeweiligen Elemente.

Ein weiterer Aspekt der Erfindung bezieht sich auf ein Verfahren, welches insbesondere mit der erfindungsgemässen Vorrichtung durchgeführt wird, zur Behandlung von Korn, insbesondere Getreide, Pseudogetreide, Ölsaaten oder Hülsenfrüchte. Zunächst erfolgt ein Befüllen eines Behälters mit Wasser und Korn. In einem anschliessenden Prozessschritt wird das Korn durch das Wasser eingeweicht. Nach Vollendung des Einweichschritts wird das Wasser aus dem Behälter entleert. Anschliessend wird in einem nächsten Prozessschritt das Korn insbesondere Zufuhr und / oder Abfuhr konditionierter Luft und / oder insbesondere unter Wenden des Korns mittels einer Wendeeinrichtung. Anschliessend wird optional als weiterer Prozessschritt das Korn gedarrt, beispielsweise auf einen Wassergehalt von 2 bis 14wt%. Als weiterer optionaler Prozessschritt insbesondere nach dem Keimen kann das Korn unter Luftabschluss zur Umgebung fermentiert werden. Die Prozessschritte Darren und Fermentieren sind in deren Abfolge austauschbar.

Ein weiterer Aspekt der Erfindung bezieht sich auf eine Verwendung der oben beschriebenen Vorrichtung zum Weichen, Keimen, Darren, Fermentieren und / oder Kombinationen daraus von Korn, insbesondere gemäss dem oben beschriebenen Verfahren, wobei die Vorrichtung insbesondere mobil ausgestaltet ist.

Im Sinne der Erfindung wird unter dem Begriff mobil verstanden, dass die erfindungsgemässe Vorrichtung mittels Schiff, Lastwagen sowie vergleichbaren Transportmitteln transportierbar ist. Bevorzugt kann die erfindungsgemässe Vorrichtung in einem ISO-Container anbringbar. Besonders bevorzugt ist das erste Vorrichtungselement ein 40-Fuss ISO-Container (Innenlänge: 12,040 Meter; Innenbreite: 2,345 Meter; Innenhöhe: 2,385 Meter) beziehungsweise ein 40-Fuss High Cube ISO-Container (Innenhöhe: 2,690 Meter im Unterschied zum 40-Fuss ISO-Container) und das zweite Vorrichtungselement ein 20-Fuss ISO-Container (Innenlänge: 5,910 Meter; Innenbreite: 2,345 Meter; Innenhöhe: 2,385 Meter).

Ein weiterer Aspekt der Erfindung bezieht sich auf die erfindungsgemässe Vorrichtung, in welcher die Platte mit mindestens einer Öffnung als Grundfläche ausgestaltet ist, dadurch gekennzeichnet, dass die Vorrichtung ein Aggregat umfasst, dadurch gekennzeichnet, dass das Aggregat zumindest eine Einrichtung zur Zufuhr und / oder Abfuhr eines insbesondere konditionierten Fluids, insbesondere für Wasser zum Weichen des Korns und / oder für ein Gas umfasst, wobei das Aggregat zur Aufnahme der erfindungsgemässen Vorrichtung, in welcher die Platte mit mindestens einer Öffnung als Grundfläche ausgestaltet ist, geeignet ist. Unter Konditionieren des Fluids wird insbesondere ein Kühlen des Gases, insbesondere beim Keimen, oder Erhitzen des Gases, insbesondere beim Darren, verstanden. Bevorzugt weist das Aggregat eine Temperiereinrichtung für das Fluid auf.

Im Weiteren kann das Aggregat nur Zufuhrleitungen und / oder Abfuhrleitungen für das Fluid umfassen und an eine weitere Versorgungseinrichtung mit einer Einrichtung zur Zufuhr und / oder Abfuhr eines Fluids anbringbar sein.

Das Aggregat kann fest installierbar und / oder mobil ausgestaltet sein.

Ein weiterer Aspekt der Erfindung bezieht sich auf ein Kit umfassend mindestens die erfindungsgemässe Vorrichtung, in welcher die Platte mit mindestens einer Öffnung als Grundfläche ausgestaltet ist, und ein Aggregat, dadurch gekennzeichnet, dass das Aggregat zumindest eine Einrichtung zur Zufuhr und / oder Abfuhr eines insbesondere konditionierten Fluids, insbesondere für Wasser zum Weichen des Korns und / oder für ein Gas umfasst, wobei das Aggregat zur Aufnahme der erfindungsgemässen Vorrichtung, in welcher eine Platte mit mindestens einer Öffnung als Grundfläche ausgestaltet ist, geeignet ist. Die Ausgestaltung eines Behälters mit einer Platte mit zumindest einer Öffnung als Grundfläche hat den Vorteil, dass das Aufnahmevolumen bei beispielsweise vorgegebenen Aussenabmessungen wie bei einem 40-Fuss ISO-Container maximiert werden kann. Zudem ist eine derartige Vorrichtung einfacher transportierbar, da das Aggregat nicht notwendigerweise mit transportiert werden muss. Beispielsweise können an zwei Orten jeweils Aggregate installiert sein, so dass entsprechend dem jeweiligen Bedarf lediglich die Vorrichtung an den jeweiligen Ort transportiert werden muss. Insbesondere wird eine Vorrichtung mit einer Platte als Grundfläche auf das Aggregat aufgesetzt, wodurch das Aggregat insbesondere an der Stelle der Grundplatte als Platte mit mindestens einer Öffnung der Vorrichtung eine dichtende Wirkung zur Umgebung hin hat.

Ein weiterer Aspekt der Erfindung bezieht sich auf ein Verfahren zur betriebsfertigen Installation einer Vorrichtung zum Weichen, Keimen, Darren, Fermentieren und Kombinationen daraus von Korn, dadurch gekennzeichnet, dass die erfindungsgemässe Vorrichtung, in welcher die Platte mit mindestens einer Öffnung als Grundfläche ausgestaltet ist, mit einem Aggregat miteinander funktional verbunden wird, dadurch gekennzeichnet, dass das Aggregat zumindest eine Einrichtung zur Zufuhr und / oder Abfuhr eines insbesondere konditionierten Fluids, insbesondere für Wasser zum Weichen des Korns und / oder für ein Gas umfasst, wobei das Aggregat zur Aufnahme einer Vorrichtung, in welcher die Platte mit mindestens einer Öffnung als Grundfläche ausgestaltet ist, geeignet ist. Die Erfindung wird nachfolgend anhand der Zeichnungen näher erläutert, ohne die Erfindung auf diese zu beschränken. Es zeigen:
- Figur 1 :: Perspektivische Darstellung einer ersten Variante der erfindungsgemässen Vorrichtung mit einem Laufsteg im ersten Vorrichtungselement
- Figur 2:: Skizzierte Seitenansicht einer erfindungsgemässen Vorrichtung
- Figur 3:: Perspektivische Darstellung einer zweiten Variante der erfindungsgemässen Vorrichtung mit einem Laufsteg ausserhalb des ersten Vorrichtungselements
- Figur 4:: Perspektivische Darstellung der erfindungsgemässen Vorrichtung mit einer Energiekettenführung mit zwei horizontal zueinander angeordneten Führungsschienen für die Energieführungseinrichtung
- Figur 5:: Perspektivische Darstellung der Wendeeinrichtung der erfindungsgemässen Vorrichtung unter der Deckfläche
- Figur 6:: Perspektivische Darstellung der Positionierung der Wendeeinrichtung in der erfindungsgemässen Vorrichtung
- Figur 7:: Perspektivische Darstellung einer dritten Variante einer einteilig ausgestalteten, erfindungsgemässen Vorrichtung
- Figur 8:: Schematische Darstellung einer Abführeinrichtung der erfindungsgemässen Vorrichtung
- Figur 9:: Perspektivische Darstellung einer vierten Variante einer erfindungsgemässen Vorrichtung mit Ausgestaltung der Platte als Grundfläche

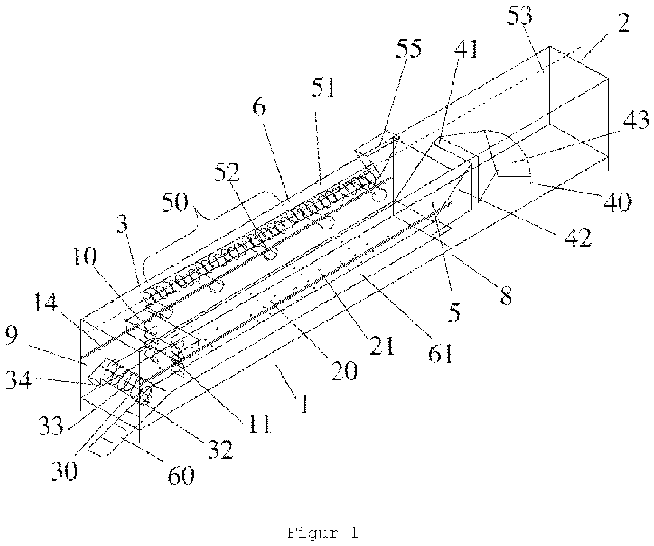

Figur 1 zeigt eine erste Variante der erfindungsgemässen Vorrichtung in perspektivischer Darstellung. Figur 1 zeigt ein erstes Vorrichtungselement 1, welches einen Behälter 3 umfasst mit einer Grundfläche 4, zwei parallelen Seitenflächen 5, zwei parallelen Stirnflächen 9 und einer Deckfläche 6. Im Weiteren zeigt Figur 1 eine Wendeeinrichtung 10 mit einer zwei Wendeschnecken 11, welche im Behälter 3 an einer Leiste 14 verschiebbar angeordnet ist. Im Weiteren ist in Figur 1 eine Platte 20 mit Öffnungen 21 horizontal beabstandet und parallel zur Grundfläche 4 an den Seitenflächen 5 und Stirnflächen 9 angebracht. Figur 1 zeigt an der Deckfläche 6 des Behälters 3 eine Verteileinrichtung 50 zur Zufuhr des zu verarbeitenden Korns entlang einer Achse 53, welche einen Aufgabetrichter 55, einen Schneckenförderer 51 als Fördereinrichtung und fünf Öffnungen 52 umfasst. Im Weiteren ist in Figur 1 eine Abfuhreinrichtung 30 mit einem konische zulaufenden Abfuhrbehälter 32 und einem im konisch zulaufenden Abfuhrbehälter angeordneten Schneckenförderer 33 sowie einer Abfuhröffnung 34 gezeigt. Figur 1 zeigt einen Laufsteg 61 im Behälter 3, welcher mittels einer Treppe 60 und einer Tür (nicht ersichtlich in Figur 1) an der Stirnfläche 9 begehbar ist. In einem zweiten Vorrichtungselement 2 dieser Variante der erfindungsgemässen Vorrichtung gemäss Figur 1 ist eine Einrichtung 40 zur Zufuhr eines Gases gezeigt, welche einen Ventilator 43, einen Wärmetauscher 41 sowie mehrere Leitbleche 42 zur Führung der Luft durch eine Öffnung (nicht ersichtlich in Figur 1) zwischen dem ersten (1) und zweiten Vorrichtungselement (2) unter die Platte 20. Im Weiteren zeigt Figur 1 ein Grundgestell 8, auf welchem das erste (1) und das zweite Vorrichtungselement (2) angebracht ist.

Von hier an und im Folgenden bezeichnen gleiche Referenzzeichen gleiche Komponenten in den Figuren.

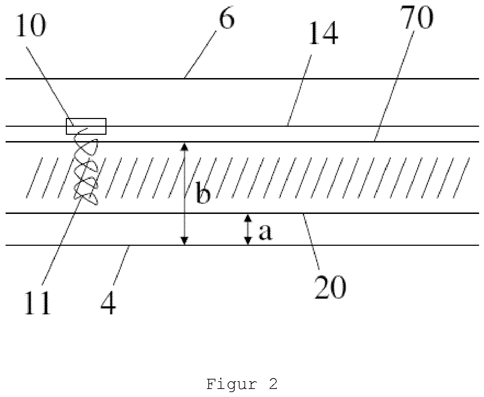

Figur 2 zeigt eine skizzierte Seitenansicht einer erfindungsgemässen Vorrichtung, wobei der Abstand a zwischen der Grundfläche 4 des Behälters 3 und der Platte 20 sowie der Abstand b zwischen der Platte 20 und der maximalen Schütthöhe 70 ersichtlich sind. In einer bevorzugten Ausführungsform der erfindungsgemässen Vorrichtung entspricht der Abstand a 465 Millimetern und der Abstand b 1'150 Millimetern.

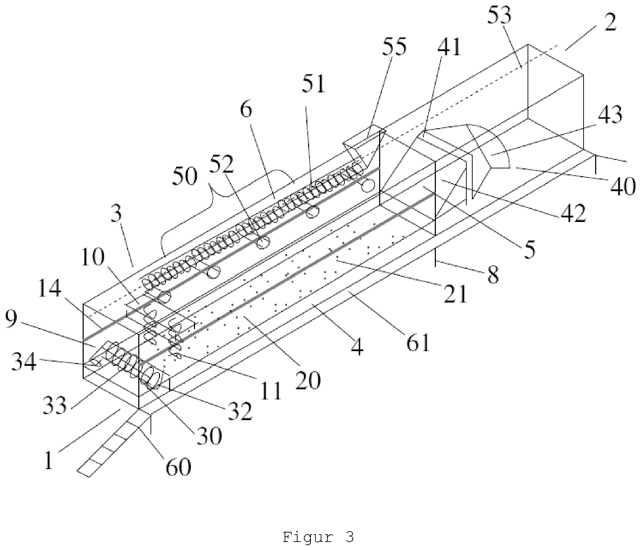

Figur 3 zeigt eine zweite Variante der erfindungsgemässen Vorrichtung in perspektivischer Darstellung. Figur 2 zeigt im Unterschied zu Figur 1 zusätzlich einen Laufsteg 61, welcher ausserhalb des Behälters 3 auf dem Grundgestell 8 angebracht ist.

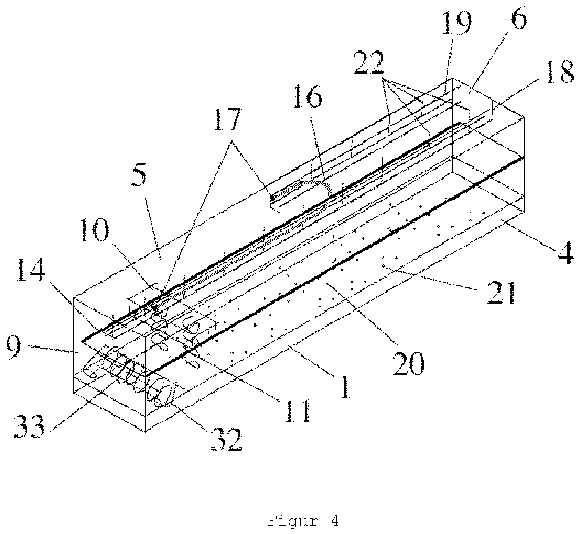

Figur 4 zeigt eine perspektivische Darstellung der erfindungsgemässen Vorrichtung mit einer Energieführungseinrichtung 16 zwischen einer ersten Führungsschiene 18 und einer zweiten Führungsschiene 19, welche im Wesentlichen parallel zueinander angeordnet und im Wesentlichen lediglich horizontal zueinander beabstandet sind, d.h. auf einer Höhe angeordnet sind. Wie in Figur 4 ersichtlich sind die beiden Führungsschienen mittels Verstrebungen 22 an der Deckfläche 6 befestigt. Im Weiteren zeigt Figur 4 eine Fixierung 17 der Energieführungskette 16 an die zweite Führungsschiene 19 sowie eine weitere Fixierung 17 der Energieführungskette 16 an die Wendeeinrichtung 10. Zudem zeigt Figur 4, dass sich die erste Führungsschiene über die Länge der Leiste 14 als Führung der Wendeeinrichtung 10 erstreckt.

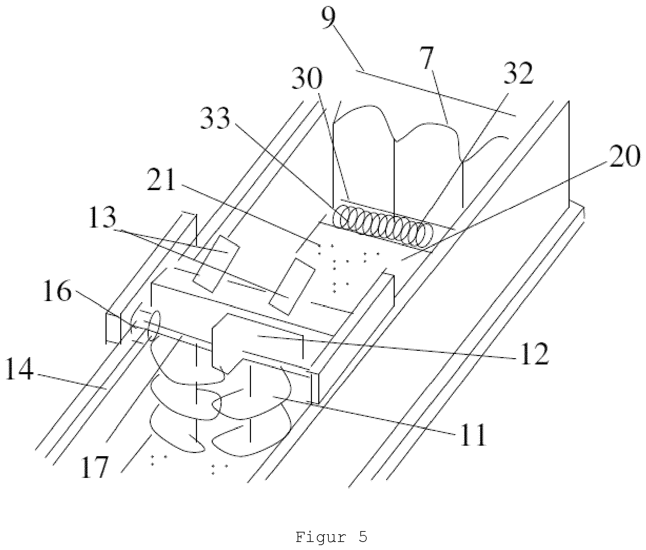

Figur 5 zeigt eine perspektivische Darstellung einer Wendeeinrichtung 10 der zweiten Variante der erfindungsgemässen Vorrichtung gemäss Figur 2 unter der Deckfläche (nicht ersichtlich in Figur 3). Figur 5 zeigt einen Antrieb 12 zum Verschieben der Wendeeinrichtung 10 mittels einer Welle 17 und einer Führungsrolle 16 entlang der Leiste 14. Im Weiteren zeigt Figur 5 zwei Antriebe 13 der Wendeschnecken 11. Zudem zeigt Figur 5 angrenzend an die Platte 20 mit Öffnungen 21 die Abfuhreinrichtung 30 umfassend den Abfuhrbehälter 32 und den Schneckenförderer 33. Figur 5 zeigt im Weiteren an der Stirnfläche 9 des Behälters 3 eine komplementäre Aussparung 7 zu den Wendeschnecken 11.

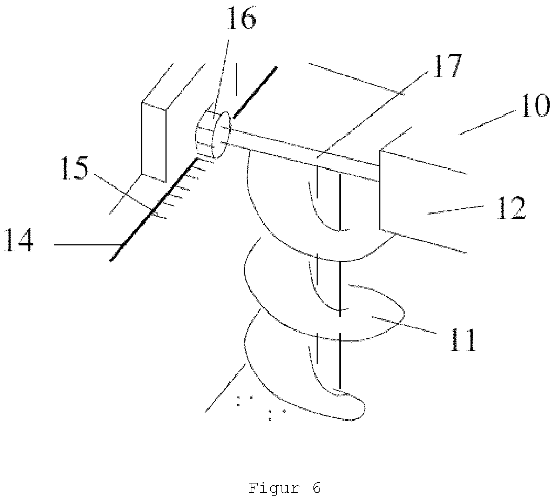

Figur 6 zeigt eine perspektivische Darstellung der Positionierung der Wendeeinrichtung 10 am Behälter 3 der erfindungsgemässen Vorrichtung. Zusätzlich zu Figur 5 wird eine Triebstockverzahnung 15 an der Leiste 14 zusammenwirkend mit der als Zahnrad ausgestalteten Führungsrolle 16 in Figur 6 gezeigt.

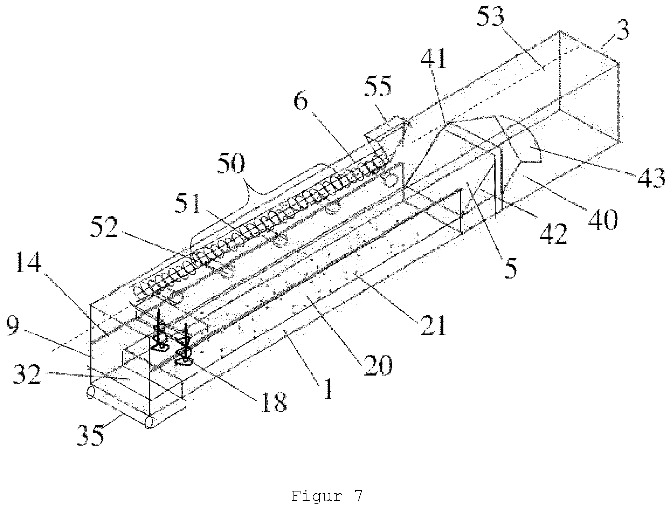

Figur 7 zeigt eine perspektivische Darstellung einer einteiligen, erfindungsgemässen Vorrichtung, wobei der Behälter 3 zusätzlich die Einrichtung 40 zur Zufuhr eines Gases mit dem Ventilator 43, dem Wärmetauscher 41 sowie mehreren Leitblechen 42 zur Führung der Luft durch eine Öffnung unter die Platte 20 umfasst. Im Behälter 3 gemäss Figur 7 wird auf Aussparungen gemäss Figur 5 an den Stirnflächen verzichtet. Figur 7 zeigt zudem die Wendeeinrichtung 10 mit zwei Wendespiralen 18, welche im Behälter 3 an der Leiste 14 verschiebbar angeordnet ist. Im Weiteren zeigt Figur 7 eine Abfuhreinrichtung 30 mit einem Abfuhrbehälter 32, welcher nicht konisch zulaufend ist und in welchem ein Förderband 35 angebracht ist. Figur 7 zeigt zudem an der Deckfläche 6 des Behälters 3 die Verteileinrichtung 50 zur Zufuhr des zu verarbeitenden Korn entlang der Achse 53, welche den Auffangtrichter 55, einen Schneckenförderer 56 als Fördereinrichtung und fünf Öffnungen 52 umfasst.

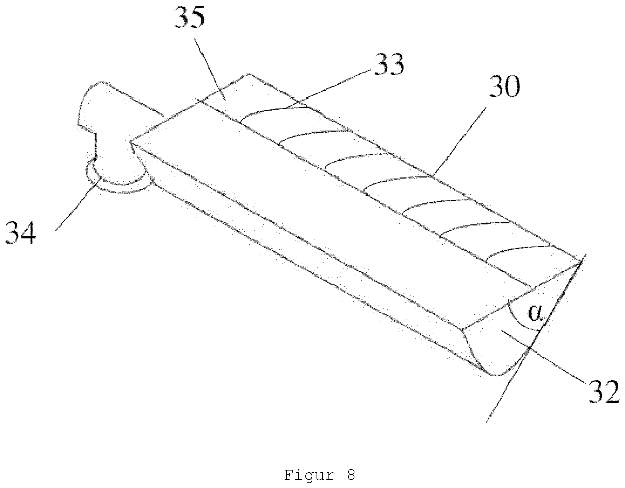

Figur 8 eine schematische Darstellung der Abfuhreinrichtung 30 einer erfindungsgemässen Vorrichtung mit Abfuhrbehälter 32, Schneckenförderer 33 sowie der Eintrittsöffnung 35. Im Weiteren ist ein Winkel α ersichtlich, welcher zwischen einer durch die Eintrittsöffnung 35 der Abfuhreinrichtung 30 gebildeten Fläche und der konischen Zulaufrichtung zum Abfuhrbehälter 32 aufgespannt wird.

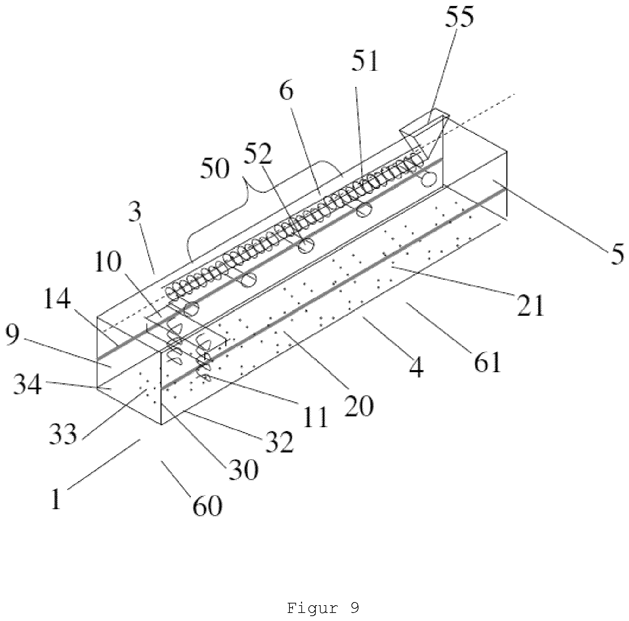

Figur 9 zeigt eine perspektivische Darstellung einer erfindungsgemässen Vorrichtung, wobei die Platte 20 mit mindestens einer Öffnung 21 als Grundfläche 4 des Behälters 3 ausgestaltet ist.

## Patentansprüche

1. Vorrichtung zum Weichen, Keimen, Darren, Fermentieren oder Kombinationen daraus von Korn, wobei die Vorrichtung einen Behälter (3) mit mindestens einer im Behälter (3) anbringbaren Platte (20) mit mindestens einer Öffnung (21) umfasst,
wobei der Behälter (3) zumindest eine im Wesentlichen rechteckige Grundfläche (4), auf der Grundfläche (4) im Wesentlichen vertikal angeordnete, zueinander parallele Seitenflächen (5), auf der Grundfläche (4) im Wesentlichen vertikal angeordnete zueinander parallele Stirnflächen (9) sowie eine auf mindestens einer Seitenfläche (5) und / oder Stirnfläche (9) im Wesentlichen horizontal angeordnete Deckfläche (6) zur Bildung eines Prozessraums umfasst und wobei auf das Innenmass bezogen eine Seitenlänge der Stirnfläche (9) 2 bis 2,5 Meter, insbesondere 2,345 Meter, entspricht.

2. Vorrichtung gemäss Anspruch 1, **dadurch gekennzeichnet, dass** die Platte (20) mit mindestens einer Öffnung (21) im Wesentlichen parallel zur Grundfläche (4) und / oder Deckfläche (6) im Behälter (3) angeordnet ist und ein Abstand (a) der Grundfläche (4) zur Platte (20) mit mindestens einer Öffnung (21) zu einem Abstand (b) der Platte mit mindestens einer Öffnung (21) zu einer maximalen Schütthöhe (71) des Korns in einem Längenverhältnis von 1 : 2 bis 1 : 3, bevorzugt 1 : 2,25 bis 1: 2,75, noch bevorzugter 1 : 2,4 bis 1: 2,5 zueinander stehen.

3. Vorrichtung gemäss Anspruch 1, **dadurch gekennzeichnet, dass** die Grundfläche (4) als Platte (20) mit mindestens einer Öffnung (21) ausgestaltet ist.

4. Vorrichtung gemäss einem der vorherigen Ansprüche, **dadurch gekennzeichnet, dass** in und / oder angrenzend an die Platte (20) mit mindestens einer Öffnung (21) eine Abfuhreinrichtung (30) zum Abführen des Korns aus dem Behälter (3) angeordnet ist sowie eine im Behälter (3) anbringbare Wendeeinrichtung (10) zum Wenden des zu verarbeitenden Korns enthalten ist, wobei die Wendeeinrichtung (10) als Einrichtung zum Fördern des Korns zur Abfuhreinrichtung (30) ausgebildet ist, insbesondere zum Entleeren des Behälters (3).

5. Vorrichtung gemäss Anspruch 4, **dadurch gekennzeichnet, dass** die Wendeeinrichtung (10) mindestens eine Wendeschnecke (11) und / oder Wendespirale, die an deren der Platte (20) zugewandtem Ende mit einem flächigen Element versehen ist, und einen Antrieb (13) für die Wendeschnecke (11) und / oder Wendespirale umfasst.

6. Vorrichtung gemäss Anspruch 4 oder 5, **dadurch gekennzeichnet, dass** die Abfuhreinrichtung (30) zumindest einen in Förderrichtung abschnittsweise konisch zulaufenden Abfuhrbehälter (32) und / oder einen, insbesondere im konisch zulaufenden Abfuhrbehälter angeordneten, Schneckenförderer (33) umfasst.

7. Vorrichtung gemäss Anspruch 6, **dadurch gekennzeichnet, dass** ein Winkel α, welcher zumindest abschnittsweise zwischen einer durch eine Eintrittsöffnung (35) der Abfuhreinrichtung (30) gebildeten Fläche und der konischen Zulaufrichtung zum Abfuhrbehälter aufgespannt wird, 10 bis 80°, bevorzugt 30 bis 70°, noch bevorzugter 50 bis 60° entspricht.

8. Vorrichtung gemäss Anspruch 4 bis 6, **dadurch gekennzeichnet, dass** die Wendeeinrichtung (10) zur Abfuhreinrichtung (30) hin bewegbar ist.

9. Vorrichtung gemäss einem der vorherigen Ansprüche, **dadurch gekennzeichnet, dass** die Seitenflächen (5) und / oder Stirnflächen (9) bei bestimmungsgemässem Gebrauch fest auf der Grundfläche (4) angeordnet sind.

10. Verfahren zur Behandlung von Korn, insbesondere Getreide, Pseudogetreide, Ölsaaten oder Hülsenfrüchte, beinhaltend mindestens die Prozessschritte:
(i) Befüllen eines Behälters mit Wasser und Korn
(ii) Weichen des Korns im Wasser
(iii) Keimen des Korn, insbesondere unter Zufuhr und / oder Abfuhr von konditionierter Luft und / oder insbesondere Wenden des Korns mittels einer Wendeeinrichtung
(iv) Optional Darren des Korns
(v) Optional Fermentieren des Korns
(vi) Entleeren des in den vorherigen Schritten behandelten Korns aus dem Behälter,
**dadurch gekennzeichnet, dass** das Verfahren durchgeführt wird in einer Vorrichtung gemäss einem der vorherigen Ansprüche.

11. Verwendung einer insbesondere mobil ausgestalteten Vorrichtung gemäss einem der Ansprüche 1 bis 9 zum Weichen, Keimen, Darren, Fermentieren oder Kombinationen daraus von Korn.

12. Vorrichtung gemäß einem der Ansprüche 3 bis 9, **dadurch gekennzeichnet, dass** die Vorrichtung ein Aggregat umfasst, **dadurch gekennzeichnet, dass** das Aggregat zumindest eine Einrichtung zur Zufuhr und / oder Abfuhr eines insbesondere konditionierten Fluids aufweist, insbesondere für Wasser zum Weichen des Korns und / oder für ein Gas, wobei das Aggregat zur Aufnahme einer Vorrichtung gemäss einem der Ansprüche 3 bis 9 geeignet ist.

13. Kit umfassend mindestens eine Vorrichtung gemäss einem der Ansprüche 3 bis 9 und ein Aggregat, **dadurch gekennzeichnet, dass** das Aggregat zumindest eine Einrichtung zur Zufuhr und / oder Abfuhr eines insbesondere konditionierten Fluids, insbesondere für Wasser zum Weichen des Korns und / oder für ein Gas umfasst, wobei das Aggregat zur Aufnahme einer Vorrichtung gemäss einem der Ansprüche 3 bis 9 geeignet ist.

14. Verfahren zur betriebsfertigen Installation einer Vorrichtung zum Weichen, Keimen, Darren, Fermentieren und Kombinationen daraus von Korn, **dadurch gekennzeichnet, dass** eine Vorrichtung gemäss einem der Ansprüche 3 bis 9 mit einem Aggregat miteinander funktional verbunden wird, **dadurch gekennzeichnet, dass** das Aggregat zumindest eine Einrichtung zur Zufuhr und / oder Abfuhr eines insbesondere konditionierten Fluids, insbesondere für Wasser zum Weichen des Korns und / oder für ein Gas umfasst, wobei das Aggregat zur Aufnahme einer Vorrichtung gemäß einem der Ansprüche 3 bis 9 geeignet ist.

## Claims

1. An apparatus for grain steeping, germinating, kiln drying, fermenting or combinations thereof, wherein the apparatus comprises a container (3) having at least one plate (20) which can be fitted in the container (3) and has at least one opening (21),
wherein the container (3) comprises at least one substantially rectangular base surface (4), side surfaces (5) being substantially vertically arranged on the base surface (4) and being parallel with respect to each other, end surfaces (9) being substantially vertically arranged on the base surface (4) and being parallel with respect to each other, as well as a top surface (6) being substantially horizontally arranged on at least one side surface (5) and/or end surface (9) for forming a process chamber, and wherein a side length of the end surface (9) corresponds to 2 to 2.5 meters, in particular 2.345 meters, with respect to the interior dimension.

2. The apparatus as claimed in claim 1, **characterized in that** the plate (20) having at least one opening (21) is arranged substantially parallel to the base surface (4) and/or top surface (6) in the container (3), and a length ratio of a distance (a) of the base surface (4) with respect to the plate (20) having at least one opening (21) with respect to a distance (b) of the plate having at least one opening (21) with respect to a maximum pouring height (71) of the grain is 1:2 to 1:3, preferably 1:2.25 to 1:2.75, more preferably 1:2.4 to 1:2.5.

3. The apparatus as claimed in claim 1, **characterized in that** the base surface (4) is configured as a plate (20) having at least one opening (21).

4. The apparatus as claimed in any of the preceding claims, **characterized in that** a removal device (30) for removing the grain from the container (3) is arranged, as well as a turning device (10) which is fittable in the container (3) and is intended for turning the grain to be processed is contained in and/or adjacent to the plate (20) having at least one opening (21), wherein the turning device (10) is designed as a device for conveying the grain to the removal device (30), in particular for emptying the container (3).

5. The apparatus as claimed in claim 4, **characterized in that** the turning device (10) comprises at least one turning screw (11) and/or turning spiral which, at its end facing the plate (20), is provided with a flat element, and a drive (13) for the turning screw (11) and/or turning spiral.

6. The apparatus as claimed in claim 4 or 5, **characterized in that** the removal device (30) comprises at least one removal container (32) tapering conically in sections in the conveying direction and/or a screw conveyor (33) arranged in particular in the conically tapering removal container.

7. The apparatus as claimed in claim 6, **characterized in that** an angle α which is opened at least in sections between a surface formed by an inlet opening (35) in the removal device (30) and the conical supply direction to the removal container is 10 to 80°, preferably 30 to 70°, more preferably 50 to 60°.

8. The apparatus as claimed in any of claims 4 to 6, **characterized in that** the turning device (10) is movable towards the removal device (30).

9. The apparatus as claimed in any of the preceding claims, **characterized in that** during intended use, the side surfaces (5) and/or end surfaces (9) are arranged fixedly on the base surface (4).

10. A method for treating grain, in particular cereals, pseudocereals, oilseeds or pulses, containing at least the following process steps:
(i) filling a container with water and grain,
(ii) steeping the grain in the water,
(iii) germinating the grain, in particular by supplying and/or removing conditioned air and/or in particular turning the grain by means of a turning device,
(iv) optionally kiln drying the grain,
(v) optionally fermenting the grain,
(vi) removing the grain treated in the previous steps from the container,
**characterized in that** the method is carried out in an apparatus as claimed in any of the preceding claims.

11. The use of an in particular mobile apparatus as claimed in any of claims 1 to 9 for grain steeping, germinating, kiln drying, fermenting or combinations thereof.

12. The apparatus as claimed in any of claims 3 to 9, **characterized in that** the apparatus comprises a unit, **characterized in that** the unit comprises at least one device for the supply and/or removal of an in particular conditioned fluid, in particular for water for steeping the grain and/or a gas, wherein the unit is suitable for receiving an apparatus as claimed in any of claims 3 to 9.

13. A kit comprising at least one apparatus as claimed in any of claims 3 to 9 and a unit, **characterized in that** the unit comprises at least one device for the supply and/or removal of an in particular conditioned fluid, in particular for water for steeping the grain and/or a gas, wherein the unit is suitable for receiving an apparatus as claimed in any of claims 3 to 9.

14. A method for the operationally ready installation of an apparatus for grain steeping, germinating, kiln drying and fermenting and combinations thereof, **characterized in that** an apparatus as claimed in any of claims 3 to 9 and a unit are functionally connected with each other, **characterized in that** the unit comprises at least one device for the supply and/or removal of an in particular conditioned fluid, in particular for water for steeping the grain and/or a gas, wherein the unit is suitable for receiving an apparatus as claimed in any of claims 3 to 9.

## Revendications

1. Dispositif pour le trempage, la germination, le touraillage, la fermentation de grains ou des combinaisons de ces opérations, ledit dispositif comprenant un récipient (3) muni d'au moins une plaque (20) pouvant être agencée dans le récipient (3) et avec au moins une ouverture (21), dans lequel le récipient (3) comprend au moins une surface de base (4) sensiblement rectangulaire, des surfaces latérales (5) parallèles disposées sensiblement verticalement sur la surface de base (4), des surfaces frontales (9) parallèles disposées sensiblement verticalement sur la surface de base (4), et une surface de recouvrement (6) disposée sensiblement horizontalement sur au moins une surface latérale (5) et/ou surface frontale (9) pour la formation d'une chambre de processus et dans lequel une longueur latérale de la surface frontale (9) par rapport à la masse interne est de 2 à 2,5 mètres, en particulier de 2,345 mètres.

2. Dispositif selon la revendication 1, **caractérisé en ce que** la plaque (20) avec au moins une ouverture (21) est disposée dans le récipient (3) sensiblement parallèlement à la surface de base (4) et/ou à la surface de recouvrement (6) et une distance (a) entre la surface de base (4) et la plaque (20) avec au moins une ouverture (21) et une distance (b) entre la plaque avec au moins une ouverture (21) et une hauteur de déversement maximale (71) des grains se situent en un rapport de longueur de 1 : 2 à 1 : 3, de préférence 1 : 2,25 à 1 : 2,75, encore plus préférentiellement de 1 : 2,4 à 1 : 2,5 l'une par rapport à l'autre.

3. Dispositif selon la revendication 1, **caractérisé en ce que** la surface de base (4) est conçue sous la forme d'une plaque (20) avec au moins une ouverture (21).

4. Dispositif selon l'une des revendications précédentes, **caractérisé en ce qu'**un système d'évacuation (30) destiné à évacuer les grains hors du récipient (3) et un système de retournement (10) pouvant être agencé dans le récipient (3) destiné à retourner les grains à transformer sont disposés dans et/ou au voisinage de la plaque (20) avec au moins une ouverture (21), le système de retournement (10) étant conçu sous la forme d'un système destiné à transporter les grains vers le système d'évacuation (30), notamment destiné à la vidange du récipient (3).

5. Dispositif selon la revendication 4, **caractérisé en ce que** le système de retournement (10) comprend au moins une vis de retournement (11) et/ou une spirale de retournement, qui est dotée d'un élément plat sur son extrémité tournée vers la plaque (20), et un entraînement (13) pour la vis de retournement (11) et/ou la spirale de retournement.

6. Dispositif selon la revendication 4 ou 5, **caractérisé en ce que** le système d'évacuation (30) comprend au moins un récipient d'évacuation (32) se terminant en forme conique sur certains tronçons dans le sens de transport et/ou un convoyeur à vis (33) disposé notamment dans le récipient se terminant en forme conique.

7. Dispositif selon la revendication 6, **caractérisé en ce qu'**un angle α, qui s'étend au moins sur certains tronçons entre une surface formée par une ouverture d'entrée (35) du système d'évacuation (30) et le sens de terminaison conique jusqu'au récipient d'évacuation, est de 10 à 80°, de préférence de 30 à 70°, encore plus préférentiellement de 50 à 60°.

8. Dispositif selon la revendication 4 à 6, **caractérisé en ce que** le système de retournement (10) est mobile jusqu'au système d'évacuation (30).

9. Dispositif selon l'une des revendications précédentes, **caractérisé en ce que** les surfaces latérales (5) et/ou les surfaces frontales (9) sont disposées fixes sur la surface de base (4) en cas d'usage conforme à la destination.

10. Procédé pour le traitement des grains, en particulier des céréales, des pseudo-céréales, des oléagineux ou des fruits à coques, comprenant au moins les étapes de processus :
(i) chargement d'un récipient avec de l'eau et des grains
(ii) trempage des grains dans l'eau
(iii) germination des grains, notamment sous apport et/ou évacuation de l'air conditionné et/ou en particulier retournement des grains au moyen d'un système de retournement
(iv) éventuellement touraillage des grains
(v) éventuellement fermentation des grains
(vi) vidange des grains traités aux étapes précédentes hors du récipient,
**caractérisé en ce que** le procédé est réalisé dans un dispositif selon l'une des revendications précédentes.

11. Utilisation d'un dispositif, en particulier de configuration mobile, selon l'une des revendications 1 à 9 pour le trempage, la germination, le touraillage, la fermentation de grains ou des combinaisons de ces opérations.

12. Dispositif selon l'une des revendications 3 à 9, **caractérisé en ce que** le dispositif comprend un groupe, **caractérisé en ce que** le groupe comprend au moins un système pour l'amenée et/ou l'évacuation d'un fluide, en particulier conditionné, en particulier pour de l'eau destinée au trempage des grains et/ou pour un gaz, le groupe étant approprié à abriter un dispositif selon l'une des revendications 3 à 9.

13. Kit comprenant au moins un dispositif selon l'une des revendications 3 à 9 et un groupe, **caractérisé en ce que** le groupe comprend au moins un système pour l'amenée et/ou l'évacuation d'un fluide, en particulier conditionné, en particulier pour de l'eau destinée au trempage des grains et/ou pour un gaz, le groupe étant approprié à abriter un dispositif selon l'une des revendications 3 à 9.

14. Procédé pour une installation prête à l'emploi d'un dispositif pour le trempage, la germination, le touraillage, la fermentation de grains ou des combinaisons de ces opérations, **caractérisé en ce qu'**un dispositif selon l'une des revendications 3 à 9 est en liaison fonctionnelle avec un groupe, **caractérisé en ce que** le groupe comprend au moins un système pour l'amenée et/ou l'évacuation d'un fluide, en particulier conditionné, en particulier pour de l'eau destinée au trempage des grains et/ou pour un gaz, le groupe étant approprié à abriter un dispositif selon l'une des revendications 3 à 9.
